# EUROPEAN PATENT SPECIFICATION

(11) **EP 4 076 177 B1**
(45) Date of publication and mention of the grant of the patent: **19.03.2025**
(21) Application number: 20902253.2
(22) Date of filing: 16.12.2020
(51) Int. Cl.: A61B 5/08, A61B 5/00, G16H 50/20, G16H 50/30, G10L 25/66, A61B 7/00, G06V 10/82, G16H 30/40, G06V 10/44, G10L 25/18, G10L 25/30

(54) **METHOD AND APPARATUS FOR AUTOMATIC COUGH DETECTION**
VERFAHREN UND VORRICHTUNG ZUR AUTOMATISCHEN HUSTENERKENNUNG
PROCÉDÉ ET APPAREIL DE DÉTECTION DE TOUX AUTOMATIQUE

(30) Priority: 16.12.2019 AU 2019904755
(43) Date of publication of application: 26.10.2022
(73) Proprietor: Pfizer Inc., New York, NY 10001-2192 (US)
(72) Inventor: WOOD, Javan Tanner, Red Hill 4059, Queensland (AU); PELTONEN, Vesa Tuomas Kristian, Red Hill 4059, Queensland (AU)
(74) Representative: HGF
(86) International application number: PCT/AU2020/051383
(87) International publication number: WO 2021/119743

(56) References cited:
- EP-B1- 1 898 786
- WO-A1-2013/142908
- WO-A2-2018/141013
- JP-A- 2009 532 072
- US-A1- 2011 087 079
- US-A1- 2016 345 893
- LIU JIA-MING ET AL: "Cough detection using deep neural networks", 2014 IEEE INTERNATIONAL CONFERENCE ON BIOINFORMATICS AND BIOMEDICINE (BIBM), IEEE, 2 November 2014 (2014-11-02), pages 560 - 563, XP032721052, DOI: 10.1109/BIBM.2014.6999220
- BARATA FILIPE ET AL: "Towards Device-Agnostic Mobile Cough Detection with Convolutional Neural Networks", 2019 IEEE INTERNATIONAL CONFERENCE ON HEALTHCARE INFORMATICS (ICHI), IEEE, 10 June 2019 (2019-06-10), pages 1 - 11, XP033663434, DOI: 10.1109/ICHI.2019.8904554

## Description

### RELATED APPLICATIONS

The present application claims priority from Australian provisional patent application No. 2019904755 filed 16 December 2019.

### TECHNICAL FIELD

The present invention relates to a method and apparatus for processing subject sounds for automatic detection of cough sounds therein.

### BACKGROUND

Any references to methods, apparatus or documents of the prior art are not to be taken as constituting any evidence or admission that they formed, or form part of the common general knowledge.

It is known to electronically process subject sounds to predict the presence of respiratory maladies. Where a symptom of the malady is coughing in the subject then it is important to be able to identify segments of the subject sounds that contain coughs, as opposed to background noise for example.

A number of approaches to identifying cough segments of patient sounds are known in the prior art. For example, in WO2013/142908 by Abeyratne at al. there is described a method for cough detection which involves determining a number of features for each of a plurality of segments of a subject's sound, forming a feature vector from those features and applying them to a pre-trained classifier. The output from the classifier is then processed to deem the segments as either "cough" or "non-cough".

A more recent approach to identifying portions of subject sounds that contain coughs is described in WO 2018/141013 (sometimes called the "LW2" method herein) in which feature vectors from the subject sound are applied to two pre-trained neural nets being respectively trained for detecting an initial phase of a cough sound and a subsequent phase of a cough sound. The first neural net is weighted in accordance with positive training to detect the initial, explosive phase, and the second neural net is positively weighted to detect one or more post-explosive phases of the cough sound. In a preferred embodiment of the LW2 method the first neural net is further weighted in accordance with positive training in respect of the explosive phase and negative training in respect of the post-explosive phases. LW2 is particularly good at identifying cough sounds in a series of connected coughs.

The Inventors have noticed that a problem that can occur with prior art cough identification methods is that they may have undesirably low specificity which means that they identify sound segments as being cough sounds when in fact they are not. Such false positive detection may make those methods infeasib le for long term use in high background noise environments where the number of non-cough events in the subject sound recording is much greater than the number of cough events.

It is known from the publication LIU JIA-MING ET AL: "Cough detection using deep neural networks",2014 IEEE INTERNATIONAL CONFERENCE ON BIOINFORMATICS AND BIOMEDICINE (BIBM), IEEE, 2 November 2014 (2014-11-02), pages 560-563, DOI: 10.1109/ BIBM.2014.6999220, a technique for detecting cough sounds using a deep neural network and for confirming the potential cough sound using a Hidden Markov Model.

It is also known according to the patent EP1898786B1, a technique for confirming a potential cough sound by using a set of four confirmatory frequencies.

It is also known from the publication BARATA FILIPE ET AL: "Towards Device-Agnostic Mobile Cough Detection with Convolutional Neural Networks",2019 IEEE INTERNATIONAL CONFERENCE ON HEALTHCARE INFORMATICS (ICHI), IEEE, 10 June 2019 (2019-06-10), pages 1-11, DOI: 10.1109/ICHI.2019.8904554, a technique for detecting a cough sound using a trained convolutional neural network using an image representation.

It would be desirable if a method and apparatus were provided that can reduce the number of false positives.

### SUMMARY OF THE INVENTION

A method for identifying cough sounds in an audio recording of a subject comprising:
operating at least one electronic processor to identify potential cough sounds in the audio recording;
operating the at least one electronic processor to transform one or more of the potential cough sounds into corresponding one or more image representations;
operating the at least one electronic processor to apply said one or more image representations to a representation pattern classifier trained to confirm that a potential cough sound is a cough sound or is not a cough sound; and
operating the at least one electronic processor to flag one or more of the potential cough sounds as confirmed cough sounds based on an output of the representation pattern classifier.

In an embodiment the method includes, operating said processor to transform the one or more sounds into the image representations wherein the image representations relate frequency and time.

In an embodiment, the one or more image representations comprise spectrograms.

In an embodiment, the one or more image representations comprise mel-spectrograms.

In an embodiment, the method includes, operating said processor to identify the potential cough sounds as cough audio segments of the audio recording by using first and second cough sound pattern classifiers trained to respectively detect initial and subsequent phases of cough sounds.

In an embodiment, the one or more image representations have a dimension of N x M pixels and are formed by said processor processing N windows of each of the cough audio segments wherein each of the N windows is analyzed in M frequency bins.

In an embodiment, each of the N windows overlaps with at least one other of the N windows.

In an embodiment, length of the windows is proportional to length of its associated cough audio segment.

In an embodiment the method includes operating said processor to calculate a Fast Fourier Transform (FFT) and a power value per frequency bin to arrive at a corresponding pixel value of the corresponding image representation of the or more image representations.

In an embodiment the method includes, operating said processor to calculate a power value per frequency bin in the form of M power values, being power values for each of the M frequency bins.

In an embodiment, the M frequency bins comprise M mel-frequency bins, the method including operating said processor to concatenate and normalize the M power values to thereby produce the corresponding image representation in the form of a mel-spectrogram image.

In an embodiment, the image representations are square and wherein M equals N.

In an embodiment, the representation pattern classifier comprises a neural network.

In an embodiment, the neural network is a convolutional neural network (CNN).

In an embodiment the method includes, operating said processor to compare a probability value comprising, or based upon, an output of the representation pattern classifier with a predetermined threshold value.

In an embodiment the method includes, operating said processor to flag one or more of the potential cough sounds as confirmed cough sounds upon the probability value exceeding the predetermined threshold value.

In an embodiment the method includes, operating said processor to flag the confirmed cough sounds by recording begin and end times of the corresponding cough audio segment as being begin and end times of a confirmed cough sound.

In an embodiment the method includes, operating said processor to generate a screen on a display responsive to said processor, the screen indicating the number of potential cough sounds processed and the number of confirmed cough sounds.

According to a further apparatus there is provided an apparatus for identifying cough sounds in a subject comprising:
an audio capture arrangement configured to store a digital audio recording of a subject in an electronic memory;
a sound segment-to-image representation assembly arranged to transform pre-identified potential cough sounds into corresponding image representations;
a representation pattern classifier in communication with the sound segment-to-image representation assembly that is configured to process the image representations to thereby produce a signal indicating a probability of the image representations corresponding to the pre-identified potential cough sounds being a confirmed cough sound.

In an embodiment the apparatus includes, or more cough sound classifiers trained to identify portions of the digital audio recording to thereby produce the pre-identified potential cough sounds.

In an embodiment, the one or more cough sound classifiers comprise a first cough sound pattern classifier and a second cough sound pattern classifiers trained to respectively detect initial and subsequent phases of cough sounds.

In an embodiment, the first cough sound pattern classifier and the and second cough sound pattern classifier each comprise neural networks.

In an embodiment, the sound segment-to-image representation assembly is arranged to transform the pre-identified potential cough sounds into corresponding image representations comprising spectrograms.

In an embodiment, the sound segment-to-image representation assembly is arranged to transform the pre-identified potential cough sounds into corresponding image representations by calculating a Fast Fourier Transform and a power per bin for M to the pre-identified potential cough sounds.

In an embodiment, the sound segment-to-image representation assembly is arranged to transform the pre-identified potential cough sounds into spectrograms

In an embodiment, the spectrograms comprise mel-spectrograms.

In an embodiment, the apparatus includes at least one electronic processor in communication with the electronic memory, wherein the processor is configured by instructions stored in the electronic memory to implement the sound segment-to-image representation assembly.

In an embodiment, the at least one electronic processor is configured by instructions stored in the electronic memory to implement the representation pattern classifier.

In an embodiment, the at least one electronic processor is configured by instructions stored in the electronic memory to implement the at least one cough sound pattern classifier arranged to identify the potential cough sounds.

According to a further aspect of the present invention there is provided a method for training a pattern classifier to confirm a potential cough sound as a confirmed cough sound from a sound recording of the subject, the method comprising:
transforming cough sounds and non-cough sounds of subjects into corresponding image representations;
training the pattern classifier to produce an output predicting that a potential cough sound is a confirmed cough sound in response to application of image representations corresponding to confirmed cough sounds and to produce an output predicting that a potential cough sound is not a cough sound in response to application of image representations corresponding to non-cough sounds.

According to another aspect there is provided a method for identifying cough sounds in an audio recording of a subject including transforming potential cough sounds in the audio recording into corresponding image representations and then applying the image representations to a pre-trained classifier and based on output from the pre-trained classifier flagging the potential cough sounds as confirmed cough sounds or not.

According to a further aspect there is provided an apparatus for processing potential cough sounds identified in an audio recording of a subject, the apparatus including at least one electronic processor in communication with a digital memory storing instructions to configure said processor to implement the method.

According to another aspect of the present invention there is provided a computer readable media bearing tangible, non-transitory machine readable instructions for one or more processors to implement a method for confirming a potential cough sound to be a confirmed cough sound based on an image representation of the potential cough sound.

### BRIEF DESCRIPTION OF THE DRAWINGS

Preferred features, embodiments and variations of the invention may be discerned from the following Detailed Description which provides sufficient information for those skilled in the art to perform the invention. The Detailed Description is not to be regarded as limiting the scope of the preceding Summary of the Invention in any way. The Detailed Description will make reference to a number of drawings as follows:
- Figure 1: is a flowchart of a cough identification method according to an embodiment of the present invention.
- Figure 2: is a block diagram of cough identification machine according to an embodiment of the present invention.
- Figure 3: is an interface screen display of the machine during recording of sounds of the subject.
- Figure 4: is a diagram illustrating a procedure of the method of the flowchart of Figure 1 for detecting potential cough sounds.
- Figure 5: is a diagram illustrating steps in the method that are implemented by the machine to produce image representations of potential cough sounds.
- Figure 6a: is a first Mel-Spectrogram image representation of a non-cough sound.
- Figure 6b: is a second Mel-Spectrogram image representation of a non-cough sound.
- Figure 7a: is a first Mel-Spectrogram image representation of a cough sound.
- Figure 7b: is a second Mel-Spectrogram image representation of a cough sound.
- Figure 8: is an interface screen display of the machine for presenting results of the cough identification method.
- Figure 9: is a block diagram of a convolutional neural network (CNN) training machine according to an embodiment of the invention.
- Figure 10: is a flowchart of a method that is coded as instructions in a software product that is executed by the training machine of Figure 9.

### DETAILED DESCRIPTION OF PREFERRED EMBODIMENTS

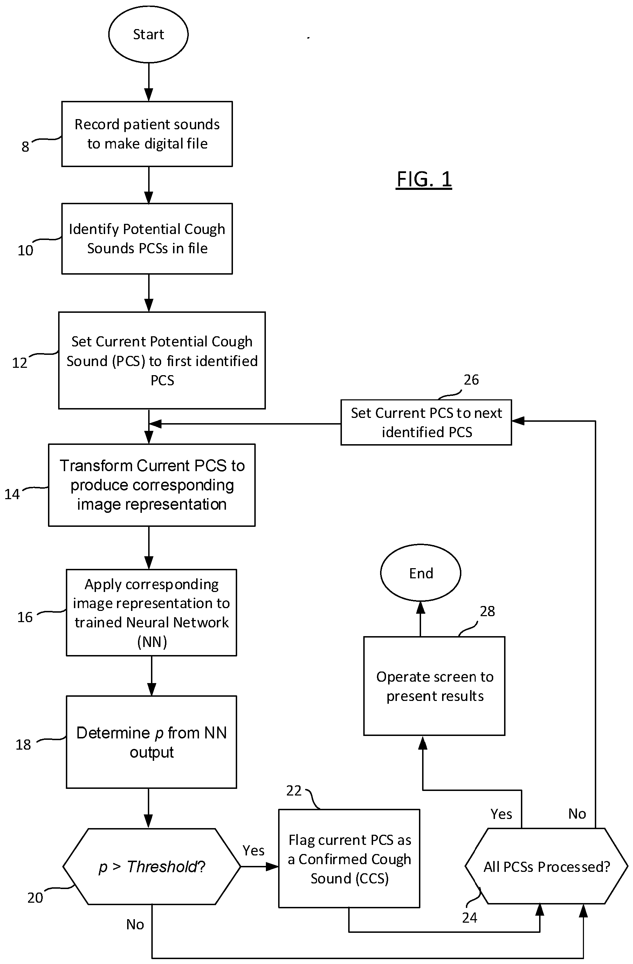

Figure 1 presents a flowchart of a method according to a preferred embodiment of the present invention for automatic cough detection.

A hardware platform that is configured to implement the method comprises a cough identification machine. The machine may be a desktop computer or a portable computational device such as a smartphone that contains at least one processor in communication with an electronic memory that stores instructions that specifically configure the processor in operation to carry out the steps of the method as will be described. It will be appreciated that it is impossible to carry out the method without the specialized hardware, i.e. either a dedicated machine or a machine that is comprised of specially programmed one or more processors. Alternatively, the machine may be implemented as a dedicated assembly that includes specific circuitry to carry out each of the steps that will be discussed. The circuitry may be largely implemented using a Field Programmable Gate Array (FPGA) configured according to a Hardware Descriptor Language (HDL) or Verilog specification.

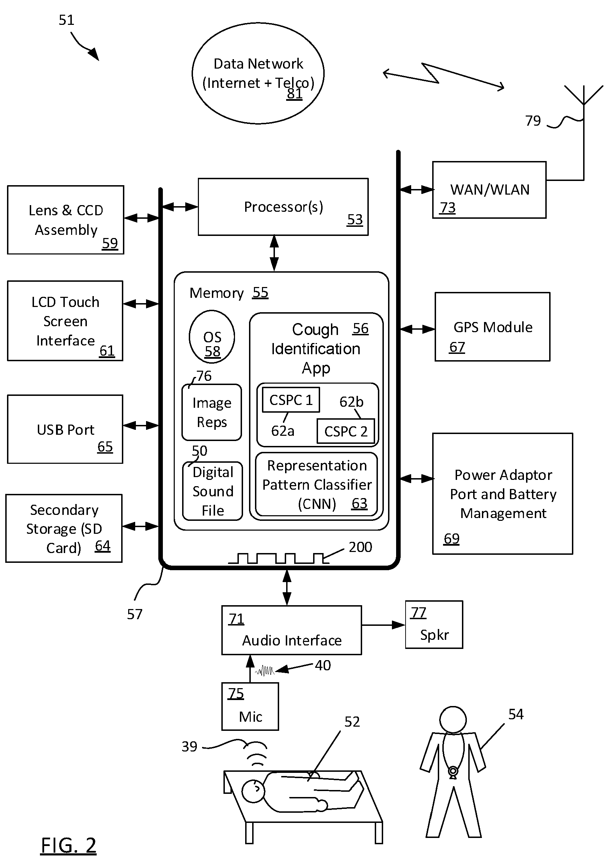

Figure 2 is a block diagram of an apparatus comprising a cough identification machine 51 that, in the presently described embodiment, is implemented using the one or more processors and memory of a smartphone. The cough identification machine 51 includes at least one processor 53 that accesses an electronic memory 55. The electronic memory 55 includes an operating system 58 such as the *Android* operating system or the *Apple iOS* operating system, for example, for execution by the processor 53. The electronic memory 55 also includes a cough identification software product or "App" 56 according to a preferred embodiment of the present invention. The cough identification App 56 includes instructions that are executable by the processor 53 in order for the cough identification machine 51 to process sounds from a subject 52 and present an identification of coughs to a clinician 54 by means of LCD touch screen interface 61. The App 56 includes instructions for the processor 53 to implement a pattern classifier such as a trained predictor or decision machine, which in the presently described preferred embodiment of the invention comprises a specially trained Convolutional Neural Network (CNN) 63.

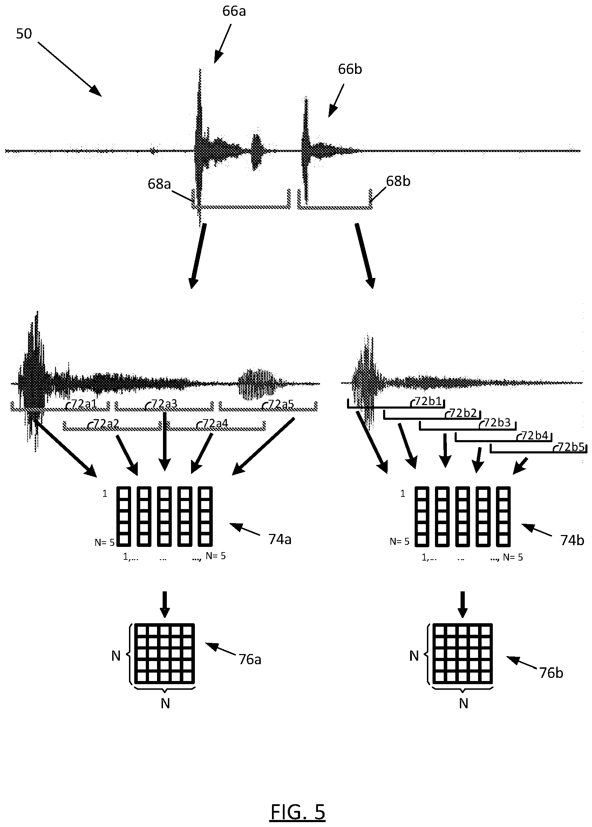

The processor 53 is in data communication with a plurality of peripheral assemblies 59 to 73, as indicated in Figure 2, via a data bus 57 which is comprised of metal conductors along which digital signals 200 are conveyed between the processor and the various peripherals. Consequently, if required the cough identification machine 51 is able to establish voice and data communication with a voice and/or data communications network 81 via WAN/WLAN assembly 73 and radio frequency antenna 79. The machine also includes other peripherals such as Lens & CCD assembly 59 which effects a digital camera so that an image of subject 52 can be captured if desired. A LCD touch screen interface 61 is provided that acts as a human-machine interface and allows the clinician 54 to read results and input commands and data into the machine 51. A USB port 65 is provided for effecting a serial data connection to an external storage device such as a USB stick or for making a cable connection to a data network or external screen and keyboard etc. A secondary storage card 64 is also provided for additional secondary storage if required in addition to internal data storage space facilitated by memory 55. Audio interface 71 couples a microphone 75 to data bus 57 and includes anti-aliasing filtering circuitry and an Analog-to-Digital sampler to convert the analog electrical waveform 40 from microphone 75 (which corresponds to subject sound wave 39) to a digital audio signal 50 (shown stored in memory in Figure 2 and shown graphically in Figure 5) that can be stored in memory 55 and processed by processor 53. The audio interface 71 is also coupled to a speaker 77. The audio interface 71 includes a Digital-to-Analog converter for converting digital audio into an analog signal and an audio amplifier that is connected to speaker 71 so that audio recorded in memory 55 or secondary storage 64 can be played back for listening by clinician 54. It will be realized that the microphone 75 and audio interface 71 along with processor 53 programmed with App 56 comprise an audio capture arrangement that is configured for storing a digital audio recording 50 of subject 52 in an electronic memory such as memory 55 or secondary storage 64.

The cough identification machine 51 is programmed with App 56 so that it is configured to operate as a machine for identifying cough segments in the recording of the subject sound.

As previously discussed, although the cough identification machine 51 that is illustrated in Figure 2 is provided in the form of smartphone hardware that is uniquely configured by App 56 it might equally make use of some other type of computational device such as a desktop computer, laptop, or tablet computational device or even be implemented in a cloud computing environment wherein the hardware comprises a virtual machine that is specially programmed with App 56. Furthermore, a dedicated cough identification machine might also be constructed that does not make use of a general purpose processor. For example, such a dedicated machine may have an audio capture arrangement including a microphone and analog-to-digital conversion circuitry configured to store a digital audio recording of the subject in an electronic memory. The machine further includes a potential cough sound identification assembly in communication with the memory and arranged to process the digital audio recording to thereby identify segments of the digital audio potentially containing cough sounds, i.e. potential cough sounds. Preferably the potential cough sound identification assembly is arranged to implement the LW2 method of WO2018/141013. A sound segment to image representation assembly may be provided that transforms identified cough sound segments into image representations. The dedicated machine further includes a hardware implemented pattern classifier to produce a signal indicating the potential cough sound as being either a confirmed cough sound or a non-cough sound.

An embodiment of the procedure that cough identification machine 51 uses to identify cough segments in a recording of subject 52, and which comprises instructions that make up App 56 is illustrated in the flowchart of Figure 1 and will now be described in detail.

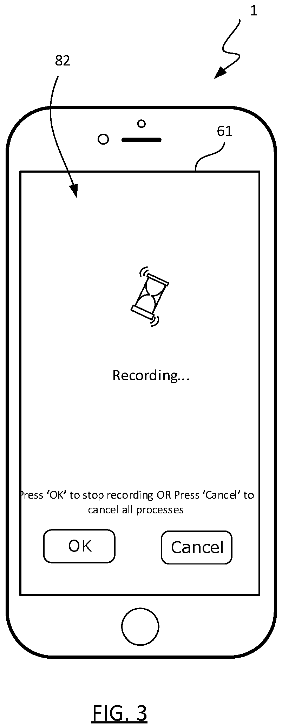

Initially clinician 54, or another carer or even subject 39, selects App 56 from an app selection screen generated by OS 58 on LCD touchscreen interface 61. In response to that selection the processor 53 displays a screen such as screen 82 of Figure 3 to prompt the clinician 54 to operate machine 51 to commence recording sound 39 from subject 52 via microphone 75 and audio interface 71. The audio interface 71 converts the sound into digital signals 200 which are conveyed along bus 57 and recorded as one or more digital files 50 by processor 53 in memory 55 and/or secondary storage SD card 64. In the presently described preferred embodiment the recording should proceed for a duration that is sufficient to include a number of cough sounds of the subject 52 to be present in the sound recording.

At box 10 processor 53 identifies potential cough sounds (PCSs) in the audio sound files 50. In a preferred embodiment of the invention the App 56 includes instructions that configure processor 53 to implement a first cough sound pattern classifier (CSPC 1) 62a and a second cough sound pattern classifier (CSPC 2) 62b, each preferably comprising neural networks trained to respectively detect initial and subsequent phases of cough sounds. Thus, in that preferred embodiment the processor 53 identifies the PCSs using the LW2 method that is described in the previously mentioned international patent application publication WO 2018/141013, the disclosure of which is hereby incorporated herein in its entirety by reference. Other methods for identifying potential cough sounds may alternatively be used at box 10, for example the methods described in the previously mentioned international patent publication WO2013/142908 by Abeyratne at al might also be used.

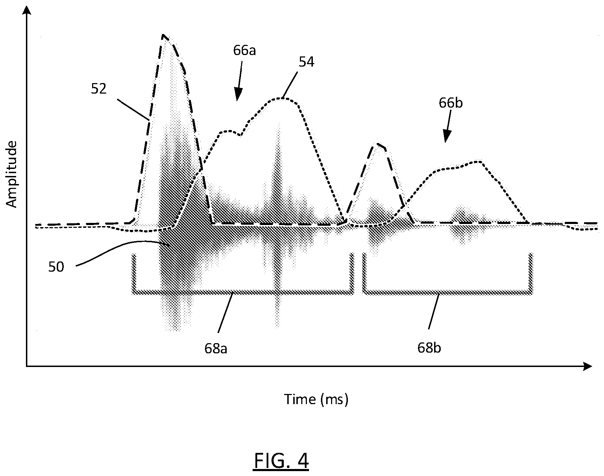

Figure 4 is a graph showing a portion of the recorded sound wave 50 from subject 52. Application of the method described in WO 2018/141013 involves applying features of the sound wave to two trained neural networks which are respectively trained to recognize a first phase and a second phase of a cough sound. The output of the first neural network is indicated as line 54 in Figure 4 and comprises a signal that represents the likelihood of a corresponding portion of the sound wave being a first phase of a cough sound. The output of the second neural network is indicated as line 52 in Figure 4 and comprises a signal that represents the likelihood of a corresponding portion of the sound wave being a subsequent phase of the cough sound. Based on the outputs 54 and 52 of the first and second trained neural networks processor 53 identifies two Potential Cough Sounds 66a and 66b which are located in segments 68a and 68b.

At box 12 the processor 53 sets a variable *Current PCS* to the first PCS that has been previously identified, i.e. "pre-identified" at box 10.

At box 14 the processor 53 transforms the pre-identified PCS that is stored in the *Current PCS* variable to produce a corresponding image representation 76 which it stores in either memory 55 or secondary storage 64.

This image representation may comprise, or be based on, a spectrogram of the Current Cough Sound portion of the digital audio file. Possible image representations include mel-frequency spectrogram (or "mel-spectrogram"), continuous wavelet transform, and derivatives of these representations along the time dimension, also known as delta features. Consequently, the image representations relate frequency, for example on a vertical axis, with time for example on a horizontal axis, over the duration of the PCS.

An example of one particular implementation of box 14 is depicted in Figure 5. Initially the processor 53 identifies two Potential Cough Sounds (PCS) 66a, 66b in the digital sound file 50.

Processor 53 identifies the Potential Cough Sounds 66a and 66b as separate cough audio segments 68a and 68b. Each of the separate cough audio segments 68a and 68b are then divided into N, in the present example N=5, equal length overlapping windows 72a1,...,72a5 and 72b1,...,72b5. For a shorter cough segment, e.g. cough segment 68b which is somewhat shorter than cough segment 68a, the overlapping windows 72b that are used to segment section 68b are proportionally shorter to the overlapping windows 72a that are used to segment section 68a.

Processor 53 then calculates a Fast Fourier Transform (FFT) and a power value per mel-bin, for M=5 bins for each of the N=5 windows, to arrive at corresponding pixel values. Machine readable instructions that configure a processor to perform these operations on the sound wave are included in App 56. Such instructions are publicly available, for example at: https://librosa.github.io/librosa/_modules/librosa/core/spectrum.html (retrieved 11 December 2019).

In the example illustrated in Figure 5, processor 53 extracts Mel-spectrograms 74a, 74b, each with M=5 Mel-frequency bins, from each of the N=5 overlapping windows 72a1,..,72a5 and 72b1,...,72b5.

Processor 53 concatenates and normalizes the values stored in the spectrograms 74a and 74b to produce corresponding Square Mel-Spectrogram images 76a and 76b representing cough sounds 66a and 66b respectively. Each of images 76a and 76b is an 8-bit greyscale MxN image where M=N.

N may be any positive integer value bearing in mind that at some N, depending on the sampling rate of the audio interface 71, the cough image will contain all information present in the original audio, which is desirable. The number of FFT bins may need to be increased to accommodate higher N.

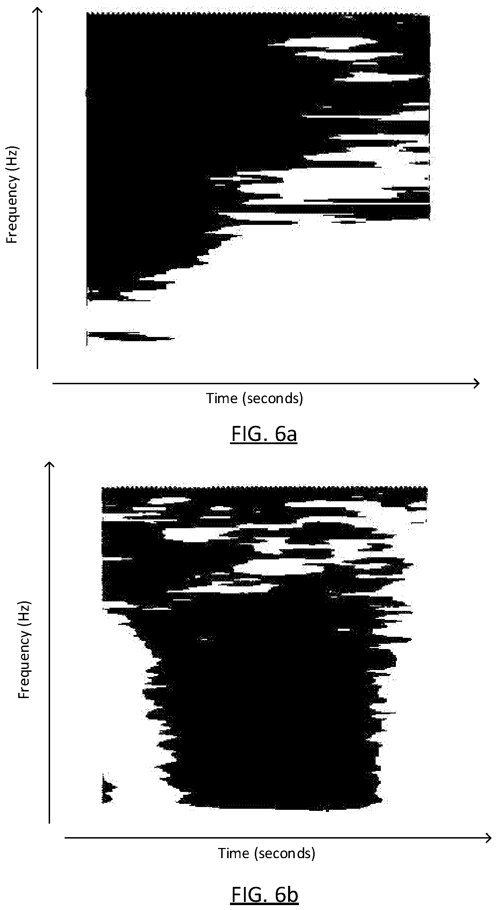

Figures 6a and 6b are Square Mel-spectrogram images of non-cough segments of the subject sound recording, obtained using the process described in Figure 5 with N = M = 224. In this image, time increases from left to right and frequency increases from bottom to top. Darker areas denote increased amplitude of the mel-frequency bin.

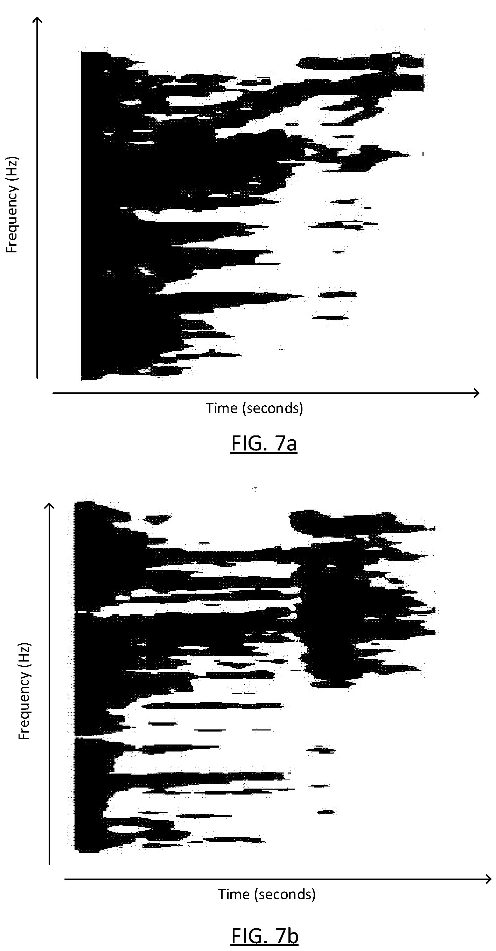

In contrast Figures 7a and 7b are Square Mel-spectrogram images of cough segments, e.g. one of segments 68a, 68b.

The images in Figures 6a to 7b have been thresholded to convert them to black and white image for purposes of official publication of this patent specification.

Although it is convenient to use square representations that are N x M pixels derived from N segments, each analyzed for M frequency bins, where N=M, it is also possible to use rectangular representations where N is not equal to M provided that the CNN 63 has been trained using similarly dimensioned rectangular images.

From the discussion of box 14 it will be understood that processor 53, configured by App 56 to perform the procedure of box 14, comprises a sound segment-to-image representation assembly that is arranged to transform sound segments of the recording, previously identified as Potential Cough Sounds, e.g. at box 10, into corresponding image representations.

Returning now to Figure 1, at box 16 processor 53 applies the image representation, for example image 76a to a representation pattern classifier in the form of the trained convolutional neural network (CNN) 63. The CNN 63 is trained to confirm whether or not the image representation of the Potential Cough Sound is indeed a cough sound i.e. a Confirmed Cough Sound (CCS). The CNN 63 comprises a representation pattern classifier that generates an output probability signal which ranges between 0 and 1 wherein 1 indicates a certainty that the Potential Cough Sound (PCS) is indeed a cough sound and thus a Confirmed Cough Sound and 0, which indicates that there is no likelihood of the PCS being a cough sound. A probability value p is arrived at box 18 from the output of the trained Neural Network (CNN) at box 16. At box 20 the p value that has been determined at box 18 is compared to a threshold value stored in variable *Threshold.* The threshold value is preferably 0.5 so that the PCS is deemed to be a CCS provided that the p value indicates that the PCS is more than likely a CCS. Higher or lower threshold values may be used as desired depending on the requirements of the specific situation.

If *p* is greater than *Threshold* at box 20 then at box 22 processor 53 flags that the current PCS is a CCS, for example by recording the corresponding sound segment's begin and end times as being the begin and end times of a confirmed cough sound (CCS).

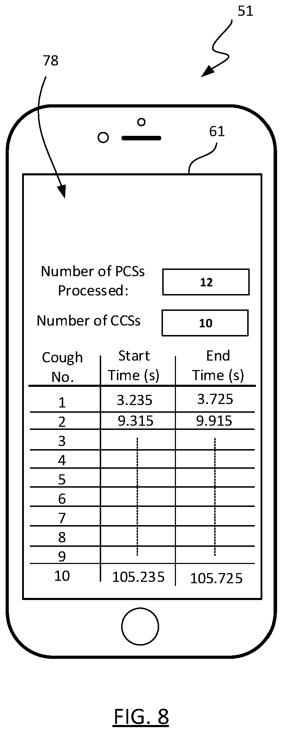

If the *p* value is not greater than *Threshold* then the PCS is not flagged as being a CCS. Control then proceeds to decision box 24. At decision box 24 processor 53 checks if there are any more PCSs to be processed. If there are more PCSs, that were identified at box 10, to be processed then at box 26 the Current PCS variable is set to the next identified PCS and control proceeds to box 14 where the previously described boxes 14 to 22 are repeated. If, at box 24, there are no more PCSs to be processed then control proceeds to box 28 where processor 53 operates a display in the form of LCD Touch Screen Interface 61, which is responsive to processor 53, to display the screen 78 shown in Figure 8. Screen 78 presents the number of PCSs processed and the number that have been found to be CCSs. It also presents the start and end times for each CCS so that clinician 54 can listen to them via speaker 77 if desired.

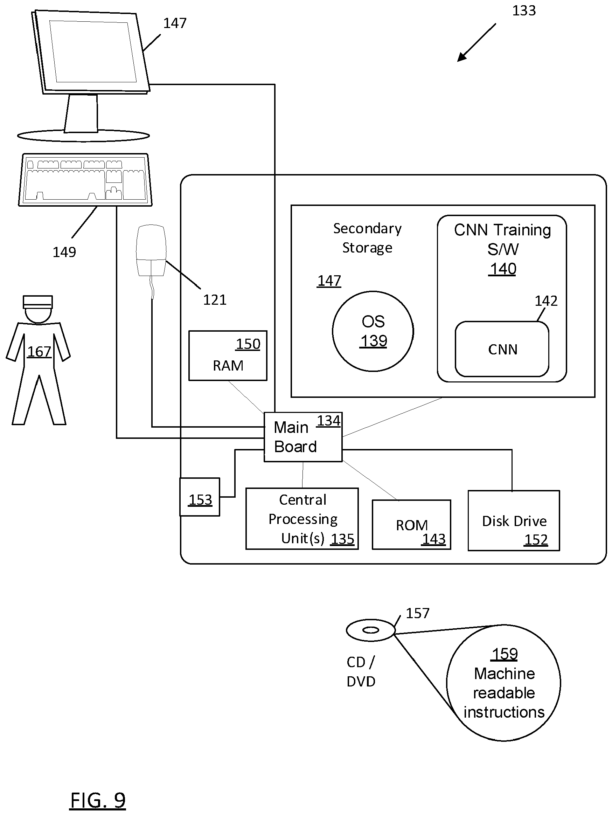

Figure 9 is a block diagram of a CNN training machine 133 implemented using the one or more processors and memory of a desktop computer configured according to CNN training Software 140. CNN training machine 133 includes a main board 134 which includes circuitry for powering and interfacing to one or more onboard microprocessors (CPUs) 135.

The main board 134 acts as an interface between microprocessors 135 and secondary memory 147. The secondary memory 147 may comprise one or more optical or magnetic, or solid state, drives. The secondary memory 147 stores instructions for an operating system 139. The main board 134 also communicates with random access memory (RAM) 150 and read only memory (ROM) 143. The ROM 143 typically stores instructions for a startup routine, such as a Basic Input Output System (BIOS) or Unified Extensible Firmware Interface (UEFI) which the microprocessor 135 accesses upon start up and which preps the microprocessor 135 for loading of the operating system 139. For example *Microsoft Windows,* and *Ubuntu* Linux Desktop are two examples of such an operating system.

The main board 134 also includes an integrated graphics adapter for driving display 147. The main board 133 will typically include a communications adapter 153, for example a LAN adaptor or a modem or a serial or parallel port, that places the server 133 in data communication with a data network.

An operator 167 of CNN training machine 133 interfaces with it by means of keyboard 149, mouse 121 and display 147.

The operator 167 may operate the operating system 139 to load software product 140. The software product 140 may be provided as tangible, non-transitory, machine readable instructions 159 borne upon a computer readable media such as optical disk 157 for reading by disk drive 152. Alternatively, it might also be downloaded via port 153.

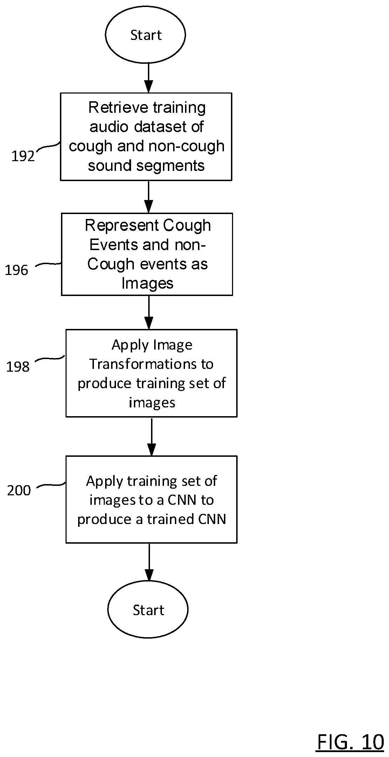

The secondary storage 147 also includes software product 140, being a CNN training software product 140 according to an embodiment of the present invention. The CNN training software product 140 is comprised of instructions for CPUs 135 (or as alternatively and collectively referred to "processor 135") to implement the method that is illustrated in Figure 10.

Initially at box 192 of Figure 10 processor 135 retrieves a training subject audio dataset which in the presently described embodiment is comprised of 70,000 cough segments and non-cough segments. The metadata includes training labels, i.e., whether or not each segment is actually a cough or not.

At box 196 the processor 135 represents the non-cough events and the cough events as images in the same manner as has previously been discussed at box 14 of Figure 1 wherein Mel-spectrogram images are created to represent each potential cough sound (PCS).

At box 198 processor 135 transforms each image produced at box 196 to create additional training examples for subsequently training a convolutional neural net (CNN). This data augmentation step at box 198 is preferable because a CNN is a very powerful learner and with a limited number of training images it can memorize the training examples and thus over fit the model. The Inventors have discerned that such a model will not generalize well on previously unseen data. The applied image transformations include, but are not limited to, small random zooming, cropping and contrast variations.

At box 200 the processor 135 trains the CNN 142 on the augmented cough and non-cough images that have been produced at box 198 and the original training labels. Over fitting of the CNN 142 is further reduced by using regularization techniques such as dropout, weight decay and batch normalization.

One example of the process used to produce a CNN 142 is to take a pretrained ResNet model, which is a residual network containing shortcut connections, such as ResNet-18, and use the convolutional layers of the model as a backbone, and replace the final non-convolutional layers with layers that suit the cough identification problem domain. These include fully connected hidden layers, dropout layers and batch normalization layers. Information about ResNet-18 is available at: https://www.mathworks.com/help/deeplearning/ref/resnet18.html (retrieved 2 December 2019), the disclosure of which is incorporated herein by reference. ResNet-18 is a convolutional neural network that is trained on more than a million images from the ImageNet database (http://www.image-net.org). The network is 18 layers deep and can classify images into 1000 object categories, such as keyboard, mouse, pencil, and many animals. As a result, the network has learned rich feature representations for a wide range of images. The network has an image input size of 224-by-224 pixels.

The Inventors have found that it is sufficient to fix the ResNet-18 layers and only train the new non-convolutional layers, however it is also possible to retrain both the ResNet-18 layers and the new non-convolutional layers to achieve a working model. A fixed dropout ratio of 0.5 is preferably used. Adaptive Moment Estimation (ADAM) is preferably used as an adaptive optimizer though other optimizer technique may also be used.

At box 202 the original (non-augmented) cough and non-cough images from box 196 are applied to the CNN 142 which is now trained to respond with probabilities for each.

The trained CNN is then distributed as CNN 63 as part of cough identification App 56 being CNN 63.

To test the performance of the method of Figure 1, the Inventors developed a dataset of 48471 coughs, and 19260 non coughs. The non-cough sounds in the dataset were specifically chosen from events that had been incorrectly flagged as a cough by the LW2 algorithm.

75% of that set was used to train the CNN 142 for Deep Cough ID and the remaining 25% (12225 coughs and 4707 non coughs) was used as a test set.

Using LW2, 12225 coughs (PCS) were identified, while 4,707 non-cough events were false positives (i.e. LW2 said these were coughs whereas further investigation revealed that they were not). When Deep Cough ID was used after LW2, 12223 coughs were identified (ie. 2 coughs were false negatives and incorrectly classified), and 4663 non-cough events were now correctly classified (rejected) and only 44 of these non-cough events were incorrectly classified as coughs.

A summary of the performance of the method of Figure 1 on the test set is set out in Table 1:

**Table 1**

| **Model** | **Segments Correctly Identified** | **Accuracy** |
|---|---|---|
| LW2 | 12225/12225 coughs | 72.2% |
| | 0/4707 non-coughs | |
| LW2+Deep Cough ID | 12123/12225 coughs | 99.14% |
| | 4663/4707 non-coughs | |

It will be observed from the above table that embodiments of the present invention result in an accuracy increase of over 25% over the prior art LW2 method that is the subject of international patent publication WO 2018/141013.

To recap, in one aspect a method is provided for identifying cough sounds, such as cough sounds 66a, 66b in an audio recording, such as digital sound file 50, of a subject 52. The method in this aspect involves operating at least one electronic processor 53 to identify potential cough sounds (box 10 of Figure 1) in the audio recording 52, for example by, but not limited to, using the LW2 procedure described in relation to Figure 4. The method also involves operating the electronic processor 53 to transform (box 14 of Figure 1) one or more of the potential cough sounds into corresponding one or more image representations such as image representations 76a, 76b (Figure 5).

The electronic processor 53 is operated to apply the one or more image representations 76a, 76b to a representation pattern classifier 63 (Figure 2) trained to confirm (box 18 of Figure 1) that a potential cough sound is a cough sound or is not a cough sound. The method includes operating the at least one electronic processor 53 to flag one or more of the potential cough sounds as confirmed cough sounds (box 22 Figure 1) based on an output of the representation pattern classifier 63.

In another aspect an apparatus has been described for identifying cough sounds in a subject. The apparatus includes an audio capture arrangement, for example comprised of microphone 75 (Figure 2) and audio interface 71 along with processor 53 configured by App 56 to capture and store a digital audio recording 50 of subject 52 in an electronic memory such as memory 55 or secondary storage 64.

The apparatus has a sound segment-to-image representation assembly arranged to transform pre-identified potential cough sounds into corresponding image representations. For example, the sound segment-to-image representation assembly may comprise processor 53, configured by App 56 to perform the procedure of box 14 (Figure 1 that is arranged to transform sound segments of the recording, previously identified as Potential Cough Sounds, e.g., at box 10, into corresponding image representations.

The apparatus also includes a representation pattern classifier in communication with the sound segment-to-image representation assembly that is configured to process the image representations to thereby produce a signal indicating a probability of the image representations corresponding to the pre-identified potential cough sounds being a confirmed cough sound. The representation pattern classifier may be in the form of a trained convolutional neural network (CNN) 63, which is trained to confirm whether or not the image representation of the Potential Cough Sound is indeed a cough sound i.e. a Confirmed Cough Sound (CCS).

In compliance with the statute, the invention has been described in language more or less specific to structural or methodical features. The term "comprises" and its variations, such as "comprising" and "comprised of" is used throughout in an inclusive sense and not to the exclusion of any additional features.

It is to be understood that the invention is not limited to specific features shown or described since the means herein described comprises preferred forms of putting the invention into effect. The invention is, therefore, claimed in any of its forms or modifications within the proper scope of the appended claims appropriately interpreted by those skilled in the art.

Throughout the specification and claims (if present), unless the context requires otherwise, the term "substantially" or "about" will be understood to not be limited to the value for the range qualified by the terms.

Any embodiment of the invention is meant to be illustrative only and is not meant to be limiting to the invention. Therefore, it should be appreciated that various other changes and modifications can be made to any embodiment described without departing from the scope of the invention defined by the appended claims.

## Claims

1. A method for identifying cough sounds in an audio recording of a subject comprising:
operating at least one electronic processor to identify potential cough sounds in the audio recording; the method being **characterised by** further comprising:
operating the at least one electronic processor to transform one or more of the potential cough sounds into corresponding one or more image representations;
operating the at least one electronic processor to apply said one or more image representations to a representation pattern classifier trained to confirm that a potential cough sound is a cough sound or is not a cough sound; and
operating the at least one electronic processor to flag one or more of the potential cough sounds as confirmed cough sounds based on an output of the representation pattern classifier.

2. The method of claim 1, including operating said processor to transform the one or more sounds into the image representations wherein the image representations relate frequency and time.

3. The method of any one of the preceding claims, wherein the one or more image representations comprise spectrograms or mel-spectrograms.

4. The method of any one of the preceding claims, including operating said processor to identify the potential cough sounds as cough audio segments of the audio recording by using first and second cough sound pattern classifiers trained to respectively detect initial and subsequent phases of cough sounds.

5. The method of claim 4, wherein the one or more image representations have a dimension of N x M pixels and are formed by said processor processing N windows of each of the cough audio segments wherein each of the N windows is analyzed in M frequency bins.

6. The method of claim 5, wherein each of the N windows overlaps with at least one other of the N windows and wherein lengths of the windows are proportional to lengths of their associated cough audio segments.

7. The method of claim 6, including operating said processor to calculate a Fast Fourier Transform (FFT) and a power value per frequency bin to arrive at a corresponding pixel value of the corresponding image representation of the or more image representations and operating said processor to calculate a power value per frequency bin in the form of M power values, being power values for each of the M frequency bins.

8. The method of claim 7, wherein the M frequency bins comprise M mel-frequency bins, the method including operating said processor to concatenate and normalize the M power values to thereby produce the corresponding image representation in the form of a mel-spectrogram image.

9. The method of any one of claims 5 to 8, wherein the image representations are square and wherein M equals N.

10. The method of any one of the preceding claims, wherein the representation pattern classifier comprises a neural network.

11. An apparatus for identifying cough sounds in a subject comprising:
an audio capture arrangement configured to store a digital audio recording of a subject in an electronic memory; the apparatus being **characterised by** further comprising:
a sound segment-to-image representation assembly arranged to transform pre-identified potential cough sounds into corresponding image representations;
a representation pattern classifier in communication with the sound segment-to-image representation assembly that is configured to process the image representations to thereby produce a signal indicating a probability of the image representations corresponding to the pre-identified potential cough sounds being a confirmed cough sound.

12. The apparatus of claim 11, including one or more cough sound classifiers trained to identify portions of the digital audio recording to thereby produce the pre-identified potential cough sounds wherein the one or more cough sound classifiers comprise a first cough sound pattern classifier and a second cough sound pattern classifiers trained to respectively detect initial and subsequent phases of cough sounds.

13. The apparatus of claim 11 or claim 12, wherein the sound segment-to-image representation assembly is arranged to transform the pre-identified potential cough sounds into corresponding image representations comprising spectrograms or mel-spectrograms.

14. The apparatus of any one of claims 11 to 13, including at least one electronic processor in communication with the electronic memory, wherein the processor is configured by instructions stored in the electronic memory to implement the sound segment-to-image representation assembly.

15. The apparatus of claim 14 , wherein the at least one electronic processor is configured by instructions stored in the electronic memory to implement at least one cough sound pattern classifier arranged to identify the potential cough sounds or is configured by instructions stored in the electronic memory to implement a first cough sound pattern classifier and a second cough sound pattern classifier arranged to identify the potential cough sounds.

## Patentansprüche

1. Verfahren zum Identifizieren von Hustengeräuschen in einer Audioaufzeichnung einer Person, umfassend:
Betreiben mindestens eines elektronischen Prozessors, um potenzielle Hustengeräusche in der Audioaufzeichnung zu identifizieren; das Verfahren **dadurch gekennzeichnet, dass** es ferner umfasst:
Betreiben des mindestens einen elektronischen Prozessors, um ein oder mehrere der potenziellen Hustengeräusche in eine oder mehrere entsprechende Bilddarstellungen umzuwandeln;
Betreiben des mindestens einen elektronischen Prozessors, um die eine oder mehreren Bilddarstellungen auf einen Darstellungsmuster-Kassifizierer anzuwenden, der darauf trainiert ist, zu bestätigen, dass ein potenzielles Hustengeräusch ein Hustengeräusch ist oder kein Hustengeräusch ist; und
Betreiben des mindestens einen elektronischen Prozessors, um ein oder mehrere der potenziellen Hustengeräusche basierend auf einer Ausgabe des Darstellungsmuster-Klassifizierers als bestätigte Hustengeräusche zu kennzeichnen.

2. Verfahren nach Anspruch 1, das den Betrieb des Prozessors einschließt, um den einen oder die mehreren Geräusche in Bilddarstellungen umzuwandeln, wobei die Bilddarstellungen Frequenz und Zeit in Beziehung setzen.

3. Verfahren nach einem der vorhergehenden Ansprüche, wobei die eine oder mehreren Bilddarstellungen Spektrogramme oder Mel-Spektrogramme umfassen.

4. Verfahren nach einem der vorhergehenden Ansprüche, das das Betreiben des Prozessors zum Identifizieren der potenziellen Hustengeräusche als Husten-Audiosegmente der Audioaufzeichnung durch Verwenden erster und zweiter Hustengeräuschmuster-Klassifizierer einschließt, die darauf trainiert sind, jeweils Anfangs- und Folgephasen von Hustengeräuschen zu erkennen.

5. Verfahren nach Anspruch 4, wobei die eine oder mehreren Bilddarstellungen eine Dimension von N x M Pixeln aufweisen und durch den Prozessor gebildet werden, der N Fenster jedes der Husten-Audiosegmente verarbeitet, wobei jedes der N Fenster in M Frequenzbereichen analysiert wird.

6. Verfahren nach Anspruch 5, wobei sich jedes der N Fenster mit mindestens einem anderen der N Fenster überlappt und wobei die Längen der Fenster proportional zu den Längen ihrer zugehörigen Husten-Audiosegmente sind.

7. Verfahren nach Anspruch 6, das das Betreiben des Prozessors zum Berechnen einer schnellen Fourier-Transformation (FFT) und eines Leistungswerts pro Frequenzbereich einschließt, um einen entsprechenden Pixelwert der entsprechenden Bilddarstellung der oder mehrerer Bilddarstellungen zu erhalten, und das Betreiben des Prozessors zum Berechnen eines Leistungswerts pro Frequenzbereich in Form von M Leistungswerten, wobei es sich um Leistungswerte für jeden der M Frequenzbereiche handelt.

8. Verfahren nach Anspruch 7, wobei die M Frequenzbereiche M Mel-Frequenzbereiche umfassen, wobei das Verfahren das Betreiben des Prozessors zum Verketten und Normalisieren der M Leistungswerte enthält, um dadurch die entsprechende Bilddarstellung in Form eines Mel-Spektrogrammbildes zu erzeugen.

9. Verfahren nach einem der Ansprüche 5 bis 8, wobei die Bilddarstellungen quadratisch sind und wobei M gleich N ist.

10. Verfahren nach einem der vorhergehenden Ansprüche, wobei der Darstellungsmuster-Kassifizierer ein neuronales Netzwerk umfasst.

11. Vorrichtung zum Identifizieren von Hustengeräuschen bei einer Person, umfassend:
eine Audio-Erfassungsanordnung, die so konfiguriert ist, dass sie eine digitale AudioAufzeichnung eines Subjekts in einem elektronischen Speicher speichert;
wobei die Vorrichtung **dadurch gekennzeichnet ist, dass** sie ferner umfasst:
eine Geräuschsegment-zu-Bilddarstellungsanordnung, die so angeordnet ist, dass sie vorab identifizierte potenzielle Hustengeräusche in entsprechende Bilddarstellungen umwandelt;
einen Darstellungsmuster-Klassifizierer in Verbindung mit der Geräuschsegment-zu-Bilddarstellungsanordnung, der so konfiguriert ist, dass er die Bilddarstellungen verarbeitet, um dadurch ein Signal zu erzeugen, das eine Wahrscheinlichkeit angibt, dass die den vorab identifizierten potenziellen Hustengeräuschen entsprechenden Bilddarstellungen ein bestätigtes Hustengeräusch sind.

12. Vorrichtung nach Anspruch 11, die einen oder mehrere Hustengeräusch-Klassifizierer beinhaltet, die darauf trainiert sind, Teile der digitalen Audioaufzeichnung zu identifizieren, um dadurch die vorab identifizierten potenziellen Hustengeräusche zu erzeugen, wobei der eine oder die mehreren Hustengeräusch-Klassifizierer einen ersten Hustengeräuschmuster-Klassifizierer und einen zweiten Hustengeräuschmuster-Klassifizierer umfassen, die darauf trainiert sind, jeweils Anfangs- und Folgephasen von Hustengeräuschen zu erkennen.

13. Vorrichtung nach Anspruch 11 oder Anspruch 12, wobei die Geräuschsegment-zu-Bilddarstellungsanordnung so ausgelegt ist, dass sie die vorab identifizierten potenziellen Hustengeräusche in entsprechende Bilddarstellungen umwandelt, die Spektrogramme oder Mel-Spektrogramme umfassen.

14. Vorrichtung nach einem der Ansprüche 11 bis 13, die mindestens einen elektronischen Prozessor in Verbindung mit dem elektronischen Speicher beinhaltet, wobei der Prozessor durch im elektronischen Speicher gespeicherte Anweisungen so konfiguriert ist, dass er die Geräuschsegment-zu-Bilddarstellungsanordnung verwirklicht.

15. Vorrichtung nach Anspruch 14, wobei der mindestens eine elektronische Prozessor durch im elektronischen Speicher gespeicherte Anweisungen so konfiguriert ist, dass er mindestens einen Hustengeräuschmuster-Klassifizierer verwirklicht, der angeordnet ist, dass er die potenziellen Hustengeräusche identifiziert, oder durch im elektronischen Speicher gespeicherte Anweisungen so konfiguriert ist, dass er einen ersten Hustengeräuschmuster-Klassifizierer und einen zweiten Hustengeräuschmuster-Klassifizierer verwirklicht, die angeordnet sind, dass sie die potenziellen Hustengeräusche identifizieren.

## Revendications

1. Procédé permettant l'identification des bruits de toux dans un enregistrement audio d'un sujet comprenant :
l'utilisation d'au moins un processeur électronique pour identifier des bruits de toux potentiels dans l'enregistrement audio ;
le procédé étant **caractérisé en ce qu'**il comprend en outre :
l'utilisation de l'au moins un processeur électronique pour transformer un ou plusieurs des bruits de toux potentiels en une ou plusieurs représentations d'image correspondantes ;
l'utilisation de l'au moins un processeur électronique pour appliquer lesdites une ou plusieurs représentations d'image à un classificateur de motifs de représentation entraîné pour confirmer qu'un bruit de toux potentiel est un bruit de toux ou n'est pas un bruit de toux ; et
l'utilisation de l'au moins un processeur électronique pour signaler un ou plusieurs des bruits de toux potentiels comme des bruits de toux confirmés sur la base d'une sortie du classificateur de motifs de représentation.

2. Procédé selon la revendication 1, comprenant l'utilisation dudit processeur pour transformer l'un ou plusieurs bruits en représentations d'image, dans lequel les représentations d'image se rapportent à la fréquence et au temps.

3. Procédé selon l'une quelconque des revendications précédentes, dans lequel l'une ou plusieurs représentations d'image comprennent des spectrogrammes ou des mel-spectrogrammes.

4. Procédé selon l'une quelconque des revendications précédentes, comprenant l'utilisation dudit processeur pour identifier les bruits de toux potentiels comme des segments audio de toux de l'enregistrement audio en utilisant des premier et second classificateurs de motifs de bruits de toux entraînés pour détecter respectivement les phases initiales et ultérieures des bruits de toux.

5. Procédé selon la revendication 4, dans lequel l'une ou plusieurs représentations d'image comportent une dimension de N x M pixels et sont formées par ledit processeur traitant N fenêtres de chacun des segments audio de toux, dans lequel chacune des N fenêtres est analysée dans M intervalles de fréquence.

6. Procédé selon la revendication 5, dans lequel chacune des N fenêtres chevauche au moins une autre des N fenêtres et dans lequel les longueurs des fenêtres sont proportionnelles aux longueurs de leurs segments audio de toux associés.

7. Procédé selon la revendication 6, comprenant l'utilisation dudit processeur pour calculer une transformée de Fourier rapide (FFT) et une valeur de puissance par intervalle de fréquence pour arriver à une valeur de pixel correspondante de la représentation d'image correspondante de l'une ou plusieurs représentations d'image et l'utilisation dudit processeur pour calculer une valeur de puissance par intervalle de fréquence sous la forme de M valeurs de puissance, étant des valeurs de puissance pour chacun des M intervalles de fréquence.

8. Procédé selon la revendication 7, dans lequel les M intervalles de fréquence comprennent M intervalles de fréquence mel, le procédé comprenant l'utilisation dudit processeur pour concaténer et normaliser les M valeurs de puissance pour produire ainsi la représentation d'image correspondante sous la forme d'une image de mel-spectrogramme.

9. Procédé selon l'une quelconque des revendications 5 à 8, dans lequel les représentations d'image sont carrées et dans lequel M est égal à N.

10. Procédé selon l'une quelconque des revendications précédentes, dans lequel le classificateur de motifs de représentation comprend un réseau neuronal.

11. Appareil permettant l'identification des bruits de toux chez un sujet comprenant :
un agencement de capture audio configuré pour stocker un enregistrement audio numérique d'un sujet dans une mémoire électronique ;
l'appareil étant **caractérisé en ce qu'**il comprend en outre :
un ensemble de représentation de segment de bruit-en-image conçu pour transformer les bruits de toux potentiels pré-identifiés en représentations d'image correspondantes ;
un classificateur de motifs de représentation en communication avec l'ensemble de représentation de segment de bruit-en-image qui est configuré pour traiter les représentations d'image afin de produire ainsi un signal indiquant une probabilité que les représentations d'image correspondant aux bruits de toux potentiels pré-identifiés soient un bruit de toux confirmé.

12. Appareil selon la revendication 11, comprenant un ou plusieurs classificateurs de bruits de toux entraînés pour identifier des parties de l'enregistrement audio numérique afin de produire ainsi les bruits de toux potentiels pré-identifiés, dans lequel l'un ou plusieurs classificateurs de bruits de toux comprennent un premier classificateur de motifs de bruits de toux et un second classificateur de motifs de bruits de toux entraînés pour détecter respectivement les phases initiales et ultérieures des bruits de toux.

13. Appareil selon la revendication 11 ou la revendication 12, dans lequel l'ensemble de représentation de segment de bruit-en-image est conçu pour transformer les bruits de toux potentiels pré-identifiés en représentations d'image correspondantes comprenant des spectrogrammes ou des mel-spectrogrammes.

14. Appareil selon l'une quelconque des revendications 11 à 13, comprenant au moins un processeur électronique en communication avec la mémoire électronique, dans lequel le processeur est configuré par des instructions stockées dans la mémoire électronique pour mettre en œuvre l'ensemble de représentation de segment de bruit-en-image.

15. Appareil selon la revendication 14, dans lequel l'au moins un processeur électronique est configuré par des instructions stockées dans la mémoire électronique pour mettre en œuvre au moins un classificateur de motifs de bruits de toux conçu pour identifier les bruits de toux potentiels ou est configuré par des instructions stockées dans la mémoire électronique pour implémenter un premier classificateur de motifs de bruits de toux et un second classificateur de motifs de bruits de toux conçus pour identifier les bruits de toux potentiels.
